# EUROPEAN PATENT APPLICATION

(11) **EP 2 347 712 A1**
(43) Date of publication of application: **27.07.2011**
(21) Application number: 09826126.6
(22) Date of filing: 12.11.2009
(51) Int. Cl.: A61B 8/00

(54) **ULTRASONOGRAPHIC DEVICE AND METHOD FOR PROCESSING SIGNAL OF ULTRASONOGRAPHIC DEVICE**

(30) Priority: 14.11.2008 JP 2008292452
(71) Applicant: Hitachi Medical Corporation, Chiyoda-ku Tokyo 101-0021 (JP)
(72) Inventor: OTAKI, Hajime, Tokyo 101-0021 (JP)
(74) Representative: MERH-IP Matias Erny Reichl Hoffmann
(86) International application number: PCT/JP2009/069267
(87) International publication number: WO 2010/055879

(57) **Abstract**

An ultrasonic diagnostic apparatus including: an ultrasonic probe configured to transmit an ultrasonic beam to an examinee, and receive a reflected echo signal from the examinee; a beam forming unit configured to supply the ultrasonic probe with a driving signal for transmitting an ultrasonic beam; a data converter configured to conduct a plurality of signal processing on echo data obtained by digitizing the reflected echo signal to convert the echo data to ultrasonic image data; and a display unit configured to display an ultrasonic image based on the converted ultrasonic image data.

## Description

### Technical Field

The present invention relates to an ultrasonic diagnostic apparatus, and particularly to a technique of enhancing the processing efficiency of a data converter for conducting signal processing on a reflected echo signal from an examinee to convert the reflected echo signal to ultrasonic image data.

### Background Art

An ultrasonic diagnostic apparatus transmits an ultrasonic wave into an inside of an examinee by an ultrasonic probe, receives a reflected echo signal from the inside of the examinee, generates various ultrasonic images such as a tomographic image (B mode image) of an imaging site of the examinee, a time variation image (M mode image) of the imaging site, a Doppler image, etc. obtained in accordance with a blood flow rate of the examinee, etc., and displays the ultrasonic images for diagnosis.

Furthermore, the ultrasonic diagnostic apparatus has many imaging modes such as a B mode for displaying a B mode image alone, a B/M mode for generating a B mode image and an M mode image through parallel processing and displaying them on the same screen, a B/D mode for generating a B mode image and a Doppler image through parallel processing and displaying them on the same screen, etc.

When plural ultrasonic images are generated by parallel processing and displayed, it is known that plural data processing systems each having a memory, a digital scan converter (DSC), etc. are provided in parallel and signal processing is executed by these data processing systems as described in Patent Document 1, for example.

### Prior Art Document

### Patent Document

Patent Document 1: JP-A-8-252253

### Summary of the Invention

### Problem to be solved by the Invention

The technique of the patent document 1 pays no attention to enhancement of the processing efficiency of a processor for executing plural signal processing different in processing rate in a process of generating and displaying an ultrasonic image.

That is, the signal processing in the ultrasonic diagnostic apparatus contains ultrasonic scan processing (for example, logarithmic compression processing, etc.) for echo data, and TV scan processing (scan conversion processing for performing coordinate-conversion to a display monitor system, etc.) on data that has been subjected to the ultrasonic scan processing, and there is a case where the rate of the ultrasonic scanning processing and the rate of the TV scan processing are different from each other.

In this case, when the signal processing having different processing rates is executed by the same processor, a processing loss occurs in the processor due to the difference in processing rate, and this is not preferable from the viewpoint of the processing efficiency.

Therefore, an object of the present invention is to enhance the processing efficiency of a processor in an ultrasonic diagnostic apparatus for executing plural signal processing different in processing rate.

### Means of solving the Problem

An ultrasonic diagnostic apparatus according to the present invention comprises an ultrasonic probe configured to transmit an ultrasonic beam to an examinee, and receive a reflected echo signal from the examinee; a beam forming unit configured to supply the ultrasonic probe with a driving signal for transmitting an ultrasonic beam; a data converter configured to conduct a plurality of signal processing on echo data obtained by digitizing the reflected echo signal to convert the echo data to ultrasonic image data; and a display unit configured to display an ultrasonic image based on the converted ultrasonic image data.

Particularly, in order to attain the object, it is characterized in that the data converter has a plurality of processors to be assigned to signal processing having different processing rates out of the plurality of signal processing.

A signal processing method for an ultrasonic diagnostic apparatus according to the present invention comprises a step that transmits an ultrasonic beam to an examinee by an ultrasonic probe, and receives a reflected echo signal from the examinee; a step that supplies the ultrasonic probe with a driving signal for transmitting the ultrasonic beam by a beam forming unit; a step that conducts a plurality of signal processing on echo data obtained by digitizing the reflected echo signal to convert the echo data to ultrasonic image data by a data converter; and a step that displays an ultrasonic image based on the converted ultrasonic image data by a display unit, wherein the data converter has a plurality of processors to be assigned to signal processing having different processing rates out of the plurality of signal processing, and the method further comprises a step for determining, from the plurality of processors, processors to be assigned to signal processing having different processing rates out of the plurality of signal processing.

According to the above construction, even when ultrasonic scan processing (logarithmic compression processing, etc.) for echo data and TV scan processing (scan conversion processing of performing coordinate conversion to display monitor system or the like) for data which has been subjected to the ultrasonic scan processing are different in processing rate, the ultrasonic scan processing and the TV scan processing can be assigned to different processors to perform a computing operation. As a result, each processor successively executes signal processing of the same processing rate, and thus signal processing having different processing rates can be executed at a high speed in parallel in a pipelining style, whereby the processing efficiency can be enhanced.

Furthermore, in accordance with an imaging mode selected from a plurality of imaging modes for ultrasonic images, the data converter can determine the number of processors to be assigned to signal processing executed in the selected imaging mode out of the plurality of processors.

That is, for example when an imaging mode is selected by a user, the data converter determines the number of necessary processors according to the content of signal processing which is set in accordance with the selected imaging mode. Here, the number of the processors may be settled in accordance with how many processors having different processing rates necessary for the selected imaging mode exist. The data converter properly selects the number of processors determined from the plurality of processors, and assigns the processors to respective signal processing.

For example, when an imaging mode for a B mode image is selected, two signal processing of the ultrasonic scan processing and the TV scan processing exist as the signal processing having the different processing rates, and thus the two processors can be used and assigned to the respective signal processing. Furthermore, when a B/M mode is selected, two signal processing having different processing rates exist for each of a B mode image and an M mode image, and thus four processors can be used and assigned to respective signal processing. When a B/D mode is selected, four processors can be likewise used and assigned to respective signal processing.

Furthermore, the data converter can vary the assignment of a plurality of processors in accordance with the beam type of an ultrasonic beam to be transmitted to the examinee. That is, the beam type of the ultrasonic beam to be transmitted to the examinee is different and the processing rate of signal processing for an reflected echo signal based on each ultrasonic beam is different every ultrasonic image to be generated and displayed. Therefore, by assigning the signal processing to different processors to be executed, whereby the processors can be efficiently used.

### Effect of the Invention

According to the present invention, the processing efficiency of the processors in the ultrasonic diagnostic apparatus which execute plural signal processing having different processing rates in parallel can be enhanced.

### Brief Description of the Drawings

[Fig. 1] Fig. 1 is a block diagram showing the construction of an ultrasonic diagnostic apparatus to which the present invention is applied.
[Fig. 2] Fig. 2 is a diagram showing the difference between a B mode image and an M mode mage of the ultrasonic diagnostic apparatus.
[Fig. 3] Fig. 3 is a diagram showing an example of the type of an ultrasonic beam used in the ultrasonic diagnostic apparatus.
[Fig. 4] Fig. 4 is a diagram showing an example of assignment of processors in generation and display of the B mode image.
[Fig. 5] Fig. 5 is a diagram showing a specific processing content of each processor in a process of generating and displaying the B mode image.
[Fig. 6] Fig. 6 is a time chart showing an executing cycle of data processing of each processor.
[Fig. 7] Fig. 7 is a diagram showing an example of assignment of processors in a B/D mode for generating and displaying a B mode image and a Doppler image in parallel.
[Fig. 8] Fig. 8 is a diagram showing a specific processing content of each processor in a process of generating and displaying the B mode image and the Doppler image.
[Fig. 9] Fig. 9 is a time chart showing an executing cycle of data processing of each processor.
[Fig. 10] Fig. 10 is a diagram showing a modification of assignment of the processors in the B/D mode for generating and displaying the B mode image and the Doppler image in parallel.
[Fig. 11] Fig. 11 is a diagram showing an example of the assignment of the processors in a B/M mode for generating and displaying a B mode image and an M mode image in parallel.
[Fig. 12] Fig. 12 is a diagram showing a modification of assignment of the processors in the B/M mode for generating and displaying the B mode image and the M mode image in parallel.
[Fig. 13] Fig. 13 is a diagram showing an example of assignment of processors in a color flow mode for generating and displaying a B mode image and a color image in parallel.
[Fig. 14] Fig. 14 is a diagram showing an example of assignment of the processors in the color flow mode for generating and displaying the B mode image and the color image in parallel.

### Modes for carrying out the Invention

Embodiments of an ultrasonic diagnostic apparatus to which the present invention is applied will be described. In the following description, parts having the same functions are represented by the same reference numerals, and the duplicative description thereof is omitted.

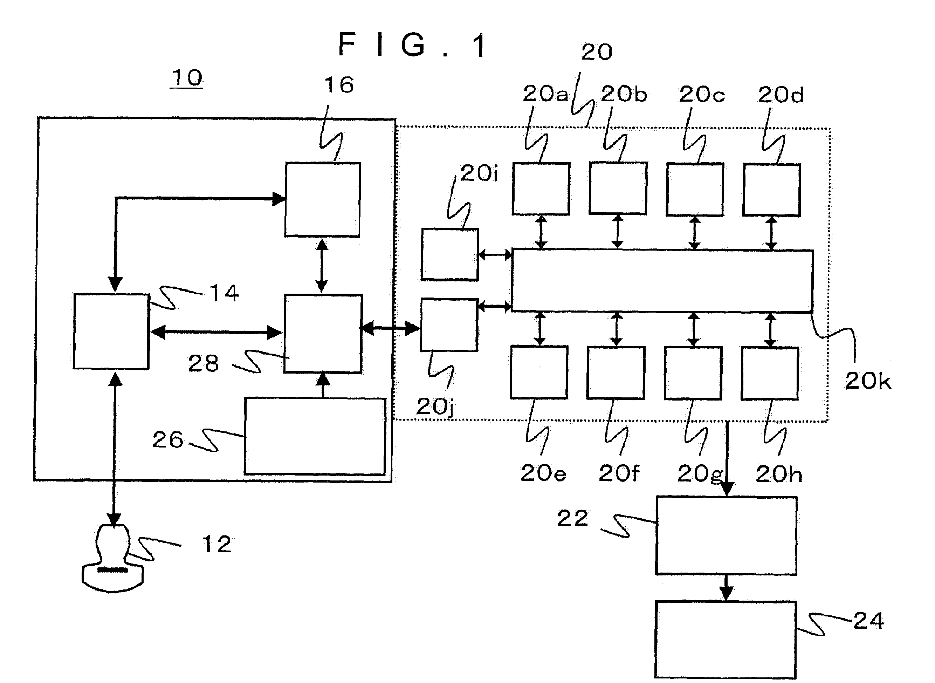

Fig. 1 is a block diagram showing the construction of an ultrasonic diagnostic apparatus to which the present invention is applied. As shown in Fig. 1, the ultrasonic diagnostic apparatus 10 according to the present invention has an ultrasonic probe 12 (PROBE) for transmitting an ultrasonic beam to an examinee, and receiving a reflected echo signal from the examinee, a transmission/reception switching unit 14 (PRB) for switching transmission/reception of the ultrasonic probe 12, a beam forming unit 16 (DBF) for supplying the ultrasonic probe 12 with a signal for transmitting an ultrasonic beam, a data converter 20 for conducting plural signal processing on echo data obtained by digitizing the reflected echo signal from the examinee to convert the echo data to ultrasonic image data, a video memory 22 for storing an ultrasonic image based on the ultrasonic image data converted by the data converter 20, etc., a display unit 24 for displaying an ultrasonic image stored in the video memory 22, etc.

Furthermore, the ultrasonic diagnostic apparatus 10 has a setting unit 26 (CONSOLE) for setting imaging mode data of the ultrasonic image data to echo data, and a controller 28 (CONT) for generating a data set by appending imaging mode data and echo data, transmitting the data set to the data converter 20, controlling the data converter 20 to analyze the imaging mode data in the data set, and converting the echo data in the data set to ultrasonic image data on the basis of the analyzed imaging mode data.

The ultrasonic probe (PROBE) has transducers of 1 to m channels which are arranged in a long axis direction of the ultrasonic probe. Here, when the ultrasonic probe is also sectioned into k parts in a short axis direction and thus transducers of 1 to k channels are arranged in the short axis direction, by varying a delay time applied to each of the transducers (1 to k channels) in the short axis direction, beam focus for wave-transmission and wave-reception can be also performed in the short axis direction. Furthermore, wave-transmission weighting is applied by changing the amplitude of an ultrasonic transmission signal supplied to each transducer in the short axis direction, and the wave-reception weighting is applied by varying the amplification degree or attenuation degree of an ultrasonic reception signal from each transducer in the short axis direction. Furthermore, aperture control can be performed by turning on/off the respective transducers in the short axis direction.

There is known the ultrasonic probe 12 having transducers which are formed of piezoelectric elements. An ultrasonic probe having transducers formed of semiconductor called as CMUT (Capacitive Micromachined Ultrasonic Transducer: IEEE Trans. Ultrason. Ferroelect. Freq. Contr. Vol 45 pp.678-690 May 1998, etc.) is applicable as the ultrasonic probe 12.

The transmission/reception switching unit 14 serves as an interface for supplying a transmission signal to the ultrasonic probe 12 and processing a received reflected echo signal. The transmission/reception switching unit 14 has the function of a wave-receiving circuit for receiving a reflected echo signal from the inside of the examinee with respect to an ultrasonic beam transmitted to the examinee to collect living body information.

The beam forming unit 16 is a wave-transmitting circuit for controlling the ultrasonic probe 12 to shoot an ultrasonic beam, and controls the transmission timing of an ultrasonic pulse for driving plural transducers of the ultrasonic probe 12 to form an ultrasonic beam toward a focus point set in the examinee. Furthermore, an ultrasonic beam is electrically scanned in the arrangement direction of the transducers of the ultrasonic probe.

The data converter 20 executes various kinds of signal processing described later on echo data which have been received by the transmission/reception switching unit 14 and then subjected to reception processing such as amplification, etc., A/D conversion, processing of adding while fitting the phase among plural transducers, etc. to convert the echo data to ultrasonic tomographic image data, and forms ultrasonic images on the basis of successively input echo data. The ultrasonic images contain an A mode image, a B mode image, a color flow mapping (C) mode image, a Doppler (D) mode image, an elasticity (elasto) (E) mode image, an M mode image, a three-dimensional ultrasonic image obtained by reconstructing B mode images which are continuously arranged mainly along the body surface of the examinee, etc.

The video memory 22 stores an ultrasonic image formed by the data converter 20, character and graphic information such as patient information, body mark information, etc. and graphic information of the setting unit 26 while combining these information. The controller 28 also has the function of a display controller for controlling selection of a display format to be selected for display.

The display unit 24 displays an ultrasonic image stored in the video memory 22, and it comprises an CRT monitor or a liquid crystal monitor, for example. The display unit 24 may be designed so that an ultrasonic image is displayed and a diagnosable image is displayed by an operator, and this invention may be applied to each of an analog output and a digital output.

The setting unit 26 is an unit through which the operator inputs various kinds of parameters such as a desired imaging mode, patient information, an imaging position, etc. by using a keyboard or a trackball on an operating table.

The controller 28 is a control computing system for controlling the transmission/reception switching unit 14, the beam forming unit 16 and the data converter 20 to function respectively on the basis of the various kinds of parameters input through the setting unit 26.

As shown in Fig. 1, the data converter 20 has plural processors 20a to 20h, a processor controller 20i for collectively controlling the processors 20a to 20h, an internal memory 20j (MEMORY) for recording echo data transmitted through the transmission/reception switching unit 14, and an internal data transmission bus 20k trough which the processors 20a to 20h, the processor controller 20i and the internal memory 20j can mutually communicate data among them.

The internal memory 20j receives and stores data transmitted from the controller 28 (CONT). The processor controller 20i controls the processors 20a to 20h connected to one another through the internal data transmission bus 20k. As a specific example of this control, data and parameters stored in the internal memory 20j are analyzed, processing programs are assigned to the processors 20a to 20h and an ultrasonic image is obtained from the data stored in the internal memory 20j.

A multiprocessor in which an architecture having plural processor-cores in a single package such as CELL or the like is standardized is applicable to the data converter 20. CELL is an abbreviation of Cell Broadband Engine (registered trademark in Japan), and a microprocessor developed by Sony Computer Entertainment Co., Ltd., etc. When CELL is used, the processors 20a to 20h are constructed as SPE (Synergistic Processor Element), the processor controller 20i is constructed as PPE (Power PC Processor Element) and the internal data transmission bus 20k is constructed as EIB (Element Interconnect Bus).

With respect to the ultrasonic diagnostic apparatus, there is a requirement of reducing the circuit scale of the data processor which contributes to miniaturization of the device, and also there is a requirement of increasing the speed of the data computing processing of the data processor which contributes to enhancement of the function of the device. Therefore, technical problems which induce tradeoff to each other have still existed. However, by adopting the multiprocessor of the simple package as described above, it can contribute to the reduction of the circuit scale and the increase of the data computing operation speed. The number of the processors is set to eight in the example of Fig. 1, however, it may be set to any number in accordance with the processing capacity of the processors. Accordingly, the number of processors may be set to any natural number.

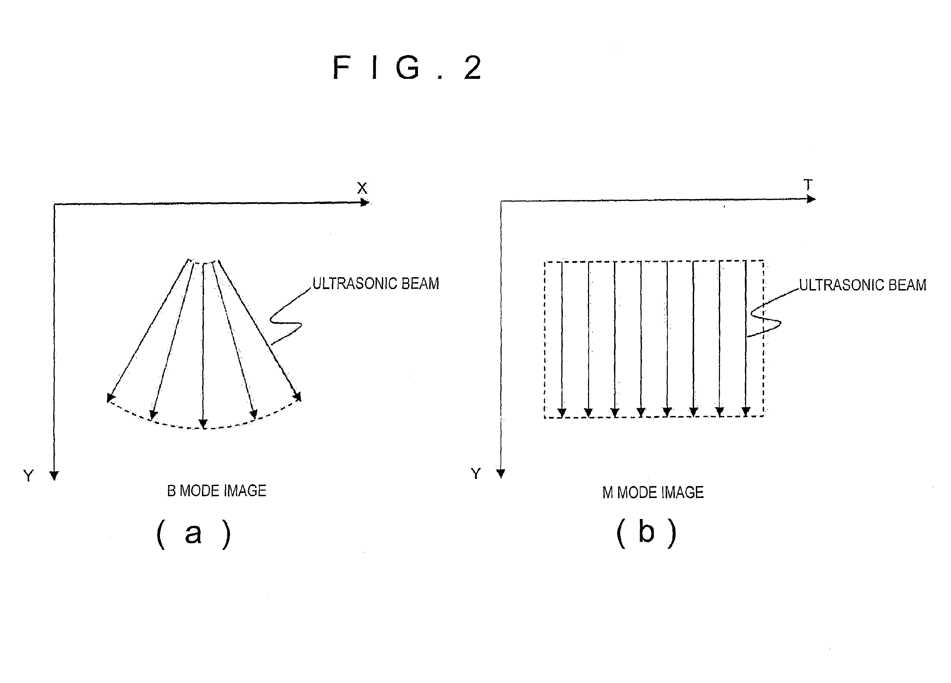

There is a case where the ultrasonic diagnostic apparatus is required to execute plural signal processing having different processing rates in a process of generating and displaying an ultrasonic image. Fig. 2 is a diagram showing the difference between the B mode image and the M mode image of the ultrasonic diagnostic apparatus as an example of the difference in processing rate which is caused by the difference of generated and displayed ultrasonic images, for example. The B mode image of Fig. 2 (a) is a two-dimensional image which is imaged by two-dimensionally scanning an ultrasonic beam. The M mode image of Fig. 2(b) is an image obtained by fixing an ultrasonic beam and displaying it with time lapse.

With respect to the B mode image, one image is generally constructed by several ultrasonic beams to several hundreds of ultrasonic beams as shown in Fig. 2(a). When the unit of this image is set as a frame, the image is updated on a frame basis. On the other hand, with respect to the M mode image, the image is updated as needed as shown in Fig. 2(b). The ultrasonic diagnostic apparatus can display the B mode image and the M mode image on the same screen at the same time. At this time, the B mode image and the M mode image can be displayed and updated asynchronously. There is a case where the processing rate is varied due to the difference in data input rate for image generation or the difference in output rate of generated images every ultrasonic image. Irrespective of the B mode image and the M mode image, there is a case where the processing rate is varied every beam type corresponding to a generated and displayed ultrasonic image as described below.

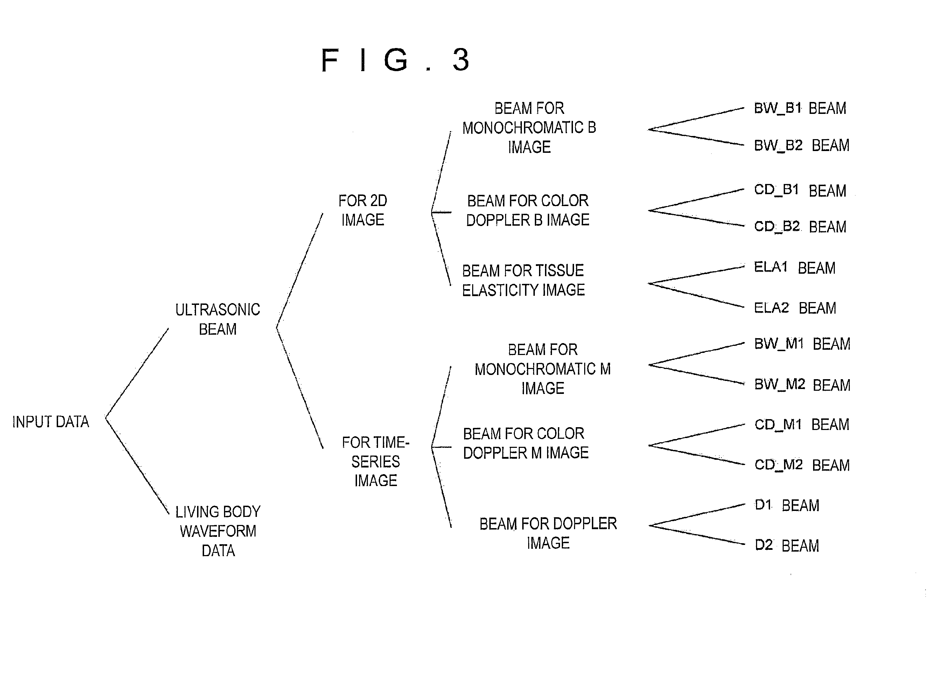

Fig. 3 is a diagram showing an example of the type of the ultrasonic beam (beam type) used in the ultrasonic diagnostic apparatus. The ultrasonic beam is mainly classified to an ultrasonic beam for 2D images and an ultrasonic beam for time-series images. The ultrasonic beam for the 2D images contains a beam for monochromatic B images, a beam for color Doppler B images, a beam for tissue elasticity images, etc., and the ultrasonic beam for the time-series images contains a beam for monochromatic M images, a beam for color Doppler images, a beam for Doppler images, etc. Furthermore, in the imaging mode of the ultrasonic diagnostic apparatus called as a biplane mode, two kinds of monochromatic B images such as a BW_B1 beam and a BW_B2 beam are required. With respect to the other ultrasonic beam types, two or more kinds of beams are likewise required due to the biplane mode.

In the example of Fig. 3, the ultrasonic diagnostic apparatus requires twelve kinds of ultrasonic beams, and when it is assumed that one processor is assigned to one kind of ultrasonic beam, twelve processors are required. However, in general, there is no imaging mode of the ultrasonic diagnostic apparatus in which twelve kinds of ultrasonic beams are simultaneously required. In the actual ultrasonic diagnostic apparatus, the beam type to be used is limited every imaging mode, and thus the ultrasonic diagnostic apparatus can be implemented by a smaller number of processors when the construction is changed every imaging mode.

Furthermore, when plural ultrasonic images are not generated and displayed in parallel, for example even when a B mode image is generated and displayed alone, there occurs a case where it is required to execute signal processing having different processing rates.
That is, in order to generate and display a B mode image, ultrasonic scan processing (for example, logarithmic compression processing, etc.) on echo data and TV scan processing (scan conversion processing of performing coordinate-conversion to display monitor system, etc.) on data which has been subjected to the ultrasonic scan processing are required, and there is a case where the rate of the ultrasonic scan processing and the rate of the TV scan processing are different from each other.

As described above, in the ultrasonic diagnostic apparatus which requires plural signal processing having different processing rates, when these signal processing is executed by the same processor, a processing loss occurs in the processor due to the difference in processing rate, and this is not preferable from the viewpoint of the processing efficiency.

Therefore, the ultrasonic diagnostic apparatus according to this embodiment is characterized in that the data converter 20 is configured to have plural processors and the signal processing having different processing rates out of plural signal processing are respectively assigned to different processors to perform the computing operation. This point will be described below by using specific embodiments.

### Embodiment 1

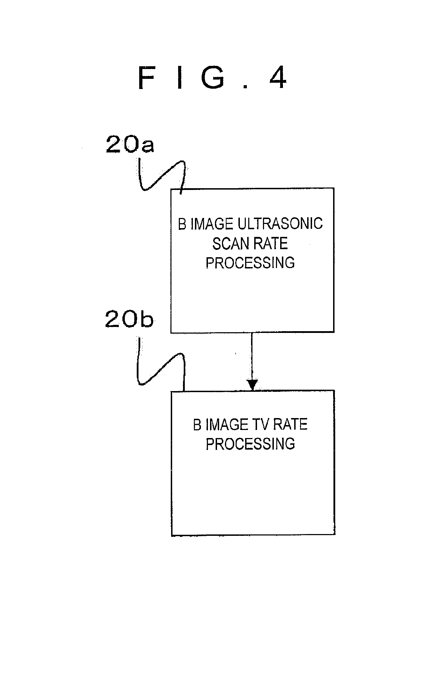

Fig. 4 is a diagram showing an example of assignment of processors to generate and display a B mode image. As shown in Fig. 4, two kinds of processing of the ultrasonic scan rate processing and the TV scan rate processing exist as signal processing having different processing rates for generation and display of B mode images. Therefore, the processor 20a (BM1) is assigned to the ultrasonic scan rate processing, and the processor 20b (BM2) is assigned to the TV scan rate processing. The assignment is not limited to the processors 20a, 20b, but any required number processors having extra processing capacities are selected from the processors 20a to 20h and assigned by the processor controller 20i.

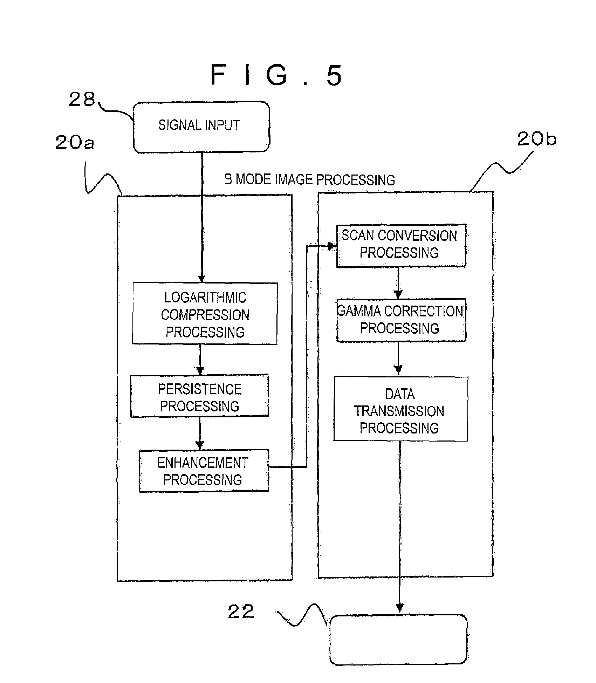

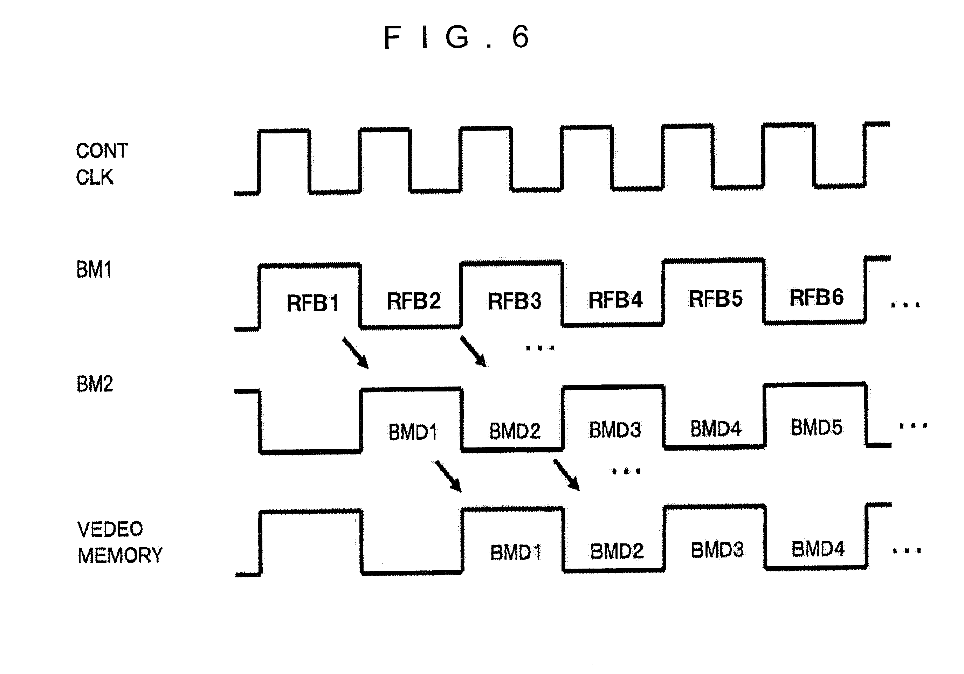

Fig. 5 is a diagram showing a specific processing content of each processor in a process of generating and displaying a B mode image, and Fig. 6 is a time chart showing the executing cycle of data processing of each processor.

As shown in Fig. 5, the processing required till a B mode image is generated and displayed roughly comprises logarithmic compression processing, persistence processing, enhancement processing, scan conversion processing, gamma correction processing and data transmission processing.

The logarithmic compression processing compresses the dynamic range of an ultrasonic reception signal of twentieth power of 2, for example, to a dynamic range on a relatively small circuit, substantially to the dynamic range of a monitor. The persistence processing adds and averages data displayed at the same position on the monitor for logarithmic-compression-processed pixels. The enhancement processing performs edge reinforcement on the persistence-processed pixels so that the boundary between pixels is clear.

The scan conversion processing subjects the enhancement-processed pixels to the coordinate-conversion from the scan of the ultrasonic beam to the scan of the display monitor. The gamma correction processing corrects display gradation for the scan-conversion-processed pixels by a gamma curved line for determining a domain of definition and a range of value for pixels. The data transmission processing transmits a gamma-correction-processed image (B mode image) to the video memory 22.

These processing can be sorted to the ultrasonic scan processing comprising the logarithmic compression processing, the persistence processing and the enhancement processing, and the TV scan processing comprising the scan conversion processing, the gamma correction processing and the data transmission processing. That is, the ultrasonic scan processing is assigned to BM1, and the TV scan processing is assigned to BM2, whereby the data of the B mode image which are successively updated through the ultrasonic scan processing can be subjected to the TV scan processing in parallel in the pipelining style.

As shown in Fig. 6, first, the processor 20a (BM1) executes the ultrasonic scan processing on RFB1 as echo data of a processing target in a first operation executing cycle (first cycle).

In the next operation executing cycle (second cycle), the processor 20b (BM2) executes the TV scan processing on RFB1 to generate B mode image data (BMD1) concerning RFB1, and outputs the B mode image data to the video memory 22. In the same pipeline operation executing cycle, the processor 20a (BM1) executes the ultrasonic scan processing on RFB2 as the next echo data to RFB1.

In the next operation executing cycle (third cycle), the video memory 22 displays BMD1 on the display unit 24. In the same pipeline operation executing cycle, the processor 20b (BM2) executes the TV scan processing on RFB2 to generate B mode image data (BMD2) concerning RFB2, and outputs the B mode image data to the video memory 22. In the same pipeline operation executing cycle, the processor 20a (BM1) executes the ultrasonic scan processing on RFB3 as the next echo data to RFB2.

In the next operation executing cycle (fourth cycle), the video memory 22 displays BMD2 on the display unit 24. In the same pipeline operation executing cycle, the processor 20b (BM2) executes the TV scan processing on RFB3, and outputs the B mode image data (BMD3) concerning BRF3 to the video memory 22. Furthermore, in the same pipeline operation executing cycle, the processor 20a (BM1) executes the ultrasonic scan processing on RFB4 as the next echo data to RFB3.

The parallel operation based on the pipelining style constructed by the three stages as described above is repeated until the B mode image data (BMDn) concerning RFBn (n represents natural number) is displayed on the display unit 24.

According to this embodiment, even when the ultrasonic scan processing (the logarithmic compression processing, the persistence processing, the enhancement processing) for the echo data and the TV scan processing (the scan conversion processing, the gamma correction processing, the data transmission processing) for the data which have been subj ected to the ultrasonic scan processing are different from each other in processing rate, the ultrasonic scan processing and the TV scan processing are assigned to different processors 20a (BM1) and 20b (BM2) to perform the operation. Accordingly, each of the processors 20a (BM1) and 20b (BM2) can successively execute signal processing having the same processing rate. Therefore, time-division processing, etc. which have been hitherto necessary for signal processing having different processing rates are not required, so that the processing loss can be prevented.

Furthermore, the ultrasonic scan processing and the TV scan processing having different processing rates can be executed in parallel at high speed in the pipelining style by using the processors 20a (BM1) and 20b (BM2), so that the processing efficiency of the data converter 20 can be enhanced.

### Embodiment 2

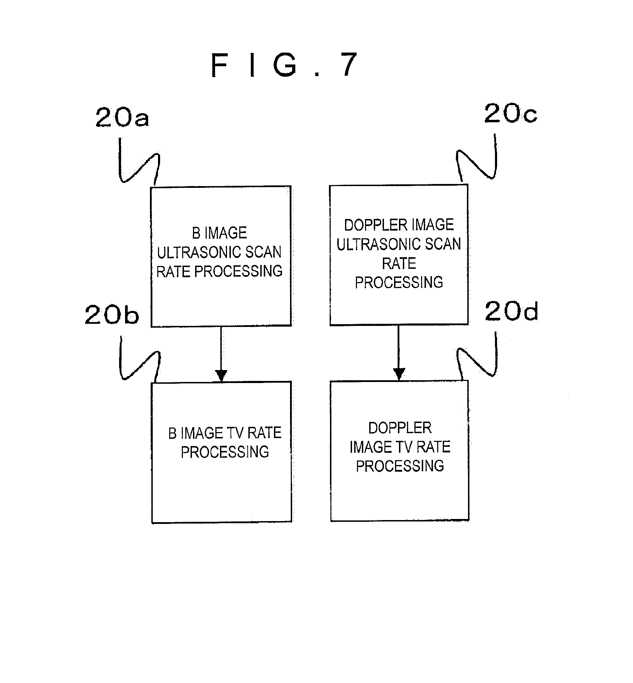

Fig. 7 is a diagram showing an example of the assignment of the processors in the B/D mode for generating and displaying a B mode image and a Doppler image in parallel. For example, when the mode is switched from the B mode to the B/D mode, the assignment of the processors 20a and 20b used in the B mode is temporarily released, and the memory area of the memory 20j secured in the B mode processing is also temporarily released. Then, in order to newly execute an operation in the B/D mode, the processors are re-assigned and a memory area to be used in the B/D mode is secured again. Here, in accordance with the selected imaging mode (B/D mode), the data converter 20 determines the number of processors to be assigned to signal processing executed in the selected imaging mode out of the processors 20a to 20h. In this embodiment, four signal processing having different processing rates exist in the signal processing required in the B/D mode, and thus four processors are assigned to the signal processing.

This embodiment provides an example in which when a B mode image and a Doppler image are alternately measured, two processors are assigned to each of the B mode image and the Doppler image in parallel to DEMUX processing for sorting data in each mode of each image, and each processor images the B mode image and the Doppler image.

As shown in Fig. 7, in the B/D mode, the two processing of the ultrasonic scan rate processing and the TV scan rate processing exist as the processing having different processing rates for each of the B mode image and the Doppler image. Therefore, the processor 20a (BM1) is assigned to the ultrasonic scan rate processing of the B mode image, and the processor 20b (BM2) is assigned to the TV scan rate processing of the B mode image. Furthermore, the processor 20c (DM1) is assigned to the ultrasonic scan rate processing of the Doppler image, and the processor 20d (DM2) is assigned to the TV scan rate processing of the Doppler image.

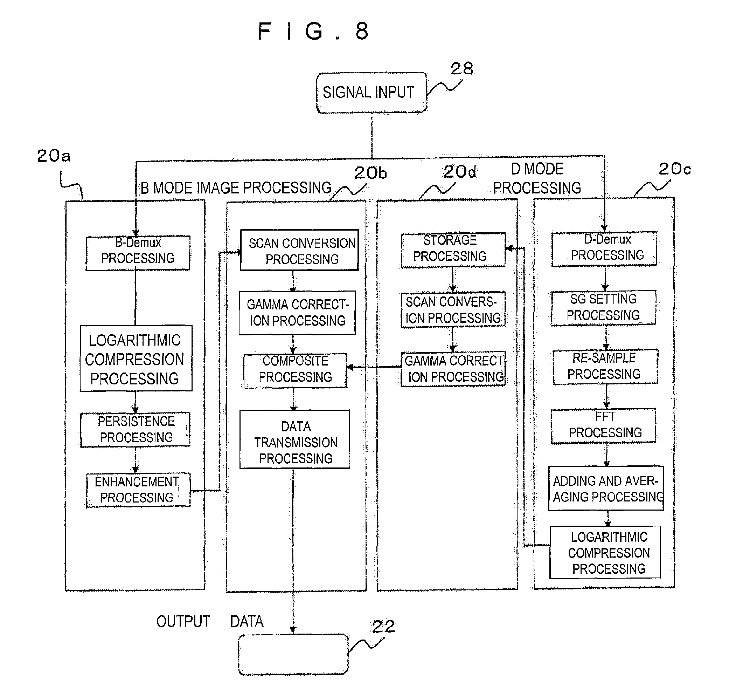

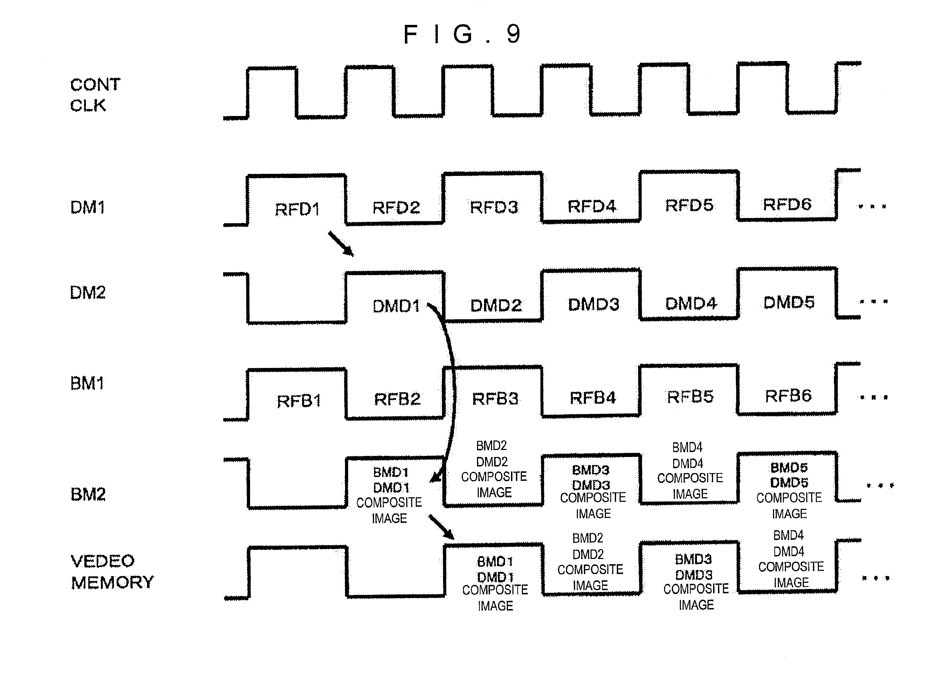

Fig. 8 is a diagram showing a specific processing content of each processor in the process of generating and displaying a B mode image and a Doppler image, and Fig. 9 is a time chart showing the executing cycle of the data processing of each processor.

The B-DEMUX processing of Fig. 8 determines whether echo data as a processing target is based on the B mode or not, and takes the processing target echo data into the processor 20a on the basis of this determination. The D-DEMUX processing determines whether the echo data as the processing target is based on the D mode, and takes the processing target echo data into the processor 20c on the basis of this determination. The determination of the echo data can be performed by analyzing data representing the beam type set incidentally to the echo data through the data converter 20.

With respect to the processing necessary to generate and display the B mode image, the description on the same parts as the first embodiment is omitted, and only different portions will be described. In the TV scan processing of the B mode image, composite processing is inserted between the gamma correction processing and the data transmission processing. The composite processing combines the B mode image and data after the TV scan processing of the Doppler image described later. Furthermore, the data transmission processing transmits the image after the composite processing (the composite image of the B mode image and the Doppler image) to the video memory 22.

The processing required until the Doppler image is generated and displayed roughly comprises sample gate (SG) setting processing, re-sample processing, Fast Fourier Transform (FFT) processing, adding and averaging processing, logarithmic compression processing, storage processing, scan conversion processing and gamma correction processing.

The SG setting processing sets SG to a desired blood flow rate diagnosis site on an ultrasonic B mode image of the examinee. The re-sample processing interpolatively determines a sample point which is calculated by Fourier Transform at the rear stage. The FFT processing executes frequency analysis on the sample point interpolated by the re-sample processing to remove clutter components of relatively low frequencies from a reflecting body having a low motion velocity such as cardiac muscle or the like and extract blood flow components of relatively high frequencies. The adding and averaging processing obtains a so-called correlation value of each sample point for the blood flow components extracted through the FFT processing. The logarithmic compression processing substantially compresses the dynamic range of the adding and averaging processing result to the dynamic range of the monitor.

The storage processing stores the logarithmic compression processing result into the internal memory 20j. The scan conversion processing subjects pixels stored in the internal memory 20j to the coordinate conversion from the scan of the ultrasonic beam to the scan of the display monitor. The gamma correction processing corrects display gradation for the scan-conversion-processed pixels by a gamma curved line for determining a domain of definition and a range of value for the pixels, and outputs the corrected display gradation to the composite processing of the B mode image.

The processing required to generate and display the Doppler image can be sorted to the ultrasonic scan processing comprising the SG set processing, the re-sample processing, the Fast Fourier Transform (FFT) processing, the adding and averaging processing and the logarithmic compression processing and the TV scan processing comprising the storage processing, the scan conversion processing and the gamma correction processing. That is, the ultrasonic scan processing is assigned to DM1, and the TV scan processing is assigned to DM2. Accordingly, the data of the Doppler image which is successively updated by the ultrasonic scan processing can be subjected to the TV scan processing in parallel in the pipelining style.

As shown in Fig. 9, in a first operation executing cycle (first cycle), the processor 20a (BM1) executes data take-in processing on RFB1 as echo data of a first processing target, and executes the ultrasonic scan processing (B mode) on RFB1. In the same pipeline operation executing cycle, the processor 20c (DM1) executes the data take-in processing on RFD1 as echo data of a processing target, and executes the ultrasonic scan processing (D mode) on RFD1.

In the next operation executing cycle (second cycle), the processor 20d (DM2) executes the TV scan processing (D mode) on RFD1 to generate Doppler image data (DMD1) concerning RFD1. The processor 20b (BM2) executes the TV scan processing on RFB1, generates the B mode image data (BMD1) concerning RFB1, and combines the B mode image data with the Doppler image data (DMD1). Furthermore, the processor 20b (BM2) transmits the B mode image data (BMD1), the Doppler image data (DMD1) and the composite image to the video memory 22. In the same pipeline operation executing cycle, the processor 20a (BM1) executes the ultrasonic scan processing (B mode) on RFB2, and the processor 20c (DM1) executes the ultrasonic scan processing (D mode) on RFD2.

In the next operation executing cycle (third cycle), the video memory 22 displays the set image out of the B mode image data (BMD1), the Doppler image data (DMD1) and the composite image on the display unit 24. In the same pipeline operation executing cycle, the processor 20d (DM2) executes the TV scan processing (D mode) on RFD2 to generate Doppler image data (DMD2) concerning RFD2. The processor 20b (BM2) executes the TV scan processing on RFB2 to generate B mode image data (BMD2) concerning RFB2, and combines the B mode image data with the Doppler image data (DMD2). Furthermore, the processor 20b (BM2) transmits the B mode image data (BMD2), the Doppler image data (DMD2) and the composite image to the video memory 22. In the same pipeline operation executing cycle, the processor 20a (BM1) executes the ultrasonic scan processing (B mode) on RFB3, and the processor 20c (DM1) executes the ultrasonic scan processing (D mode) on RFD3.

Subsequently, the parallel operation based on the pipelining style which is constructed by five stages is repeated until the B mode image data (BMDn) concerning RFBn (n represents natural number), the Doppler image data (DMDn) concerning RFDn (n represents natural number) and the composite image of BMDn and DMDn are displayed on the display unit 24.

As described above, according to this embodiment, with respect to each of the B mode image and the Doppler image, the ultrasonic scan processing for the echo data and the TV scan processing for the data which has been subjected to the ultrasonic scan processing are assigned to the different processors 20a (BM1), 20b (BM2), 20c (DM1) and 20d (DM2) to perform the operation. As a result, each of the processors 20a (BM1), 20b (BM2) , 20c (DM1) and 20d (DM2) can successively execute the signal processing having the same processing rate, and thus the processing loss can be prevented.

Furthermore, the ultrasonic scan processing and the TV scan processing which are different in processing rate can be executed at a high speed in parallel in the pipelining style by using the processors 20a (BM1), 20b (BM2), 20c (DM1) and 20d (DM2), and thus the processing efficiency of the data converter 20 can be enhanced.

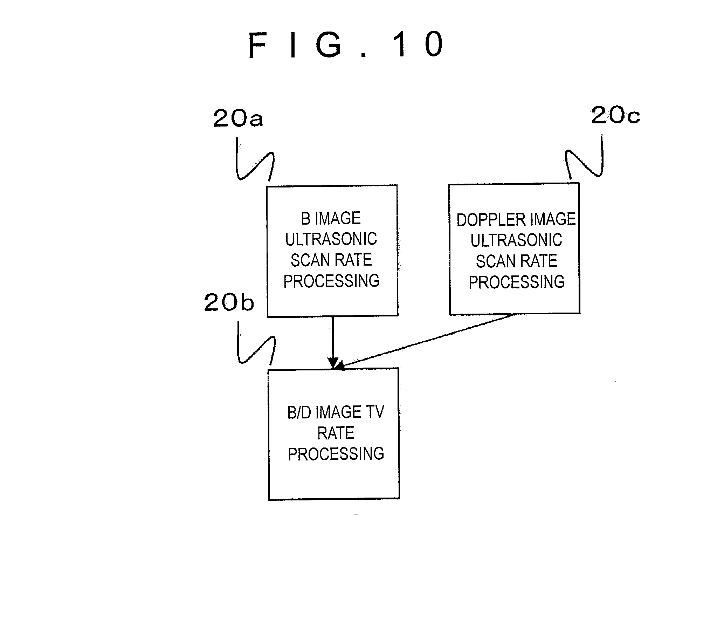

Fig. 10 is a diagram showing a modification of this embodiment. For example when the processing capacity of one processor is large, the TV scan processing for the B mode image and the TV scan processing for the Doppler image may be assigned to one processor 20b as shown in Fig. 10. That is, the processing rate of the ultrasonic scan processing is different between the B mode image and the Doppler image, and thus the different processors 20a and 20c are assigned to them. However, the processing rate of the TV scan processing is equal between both the images, and thus the processor 20b can be commonly assigned to both the images when the processor has an extra processing capacity.

### Embodiment 3

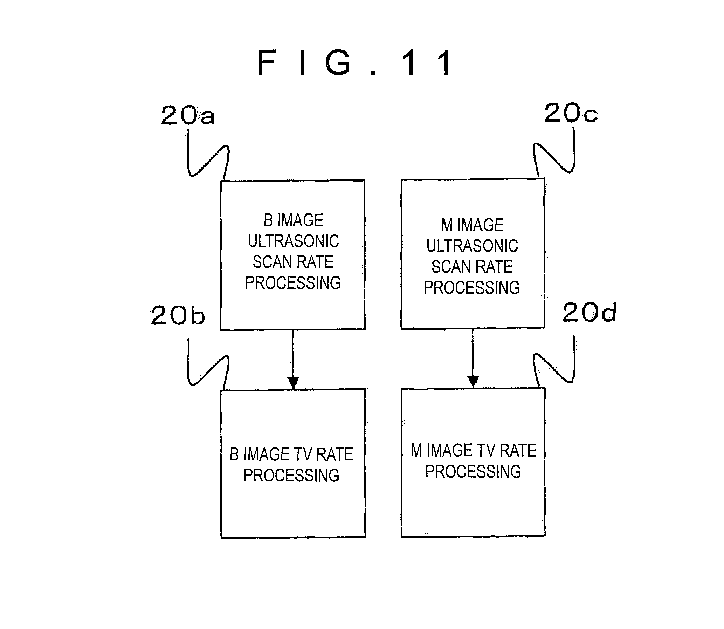

Fig. 11 is a diagram showing an example of the assignment of the processors in the B/M mode for generating and displaying the B mode image and the M mode image in parallel. For example, when the mode is switched from the B/D mode to the B/M mode, the assignment of the processors 20a to 20d used in the B/D mode is temporarily released, and the memory area of the memory 20j secured in the B/D mode processing is temporarily released. Then, in order to newly perform the operation of the B/M mode, the processors are re-assigned and a memory area used in the B/M mode is secured again. Here, the data converter 20 determines the number of processors to be assigned to the signal processing executed in the selected imaging mode out of the processors 20a to 20h in accordance with the selected imaging mode (B/M mode). In this embodiment, four signal processing having different processing rates exist as the signal processing required in the B/M mode, and thus four processors are assigned to the signal processing.

This embodiment relates to an example in which the B mode image and the M mode image are alternately measured, two processors are assigned to each of the B mode image and the M mode image in parallel to the DEMUX processing for sorting the data of each mode of each image, and each processor images the B mode image and the M mode image.

As shown in Fig. 11, in the B/M mode, two processing of the ultrasonic scan rate processing and the TV scan rate processing exist as the signal processing having the different processing rates for each of the B mode image and the M mode image as in the case of the second embodiment. Therefore, the processor 20a (BM1) is assigned to the ultrasonic scan rate processing of the B mode image, and the processor 20b (BM2) is assigned to the TV scan rate processing of the B mode image. Furthermore, the processor 20c (MM1) is assigned to the ultrasonic scan rate processing of the M mode image, and the processor 20d (MM2) is assigned to the TV scan rate processing of the M mode image.

In this embodiment, the data processing of each processor is executed as in the case of the second embodiment except that the processing required to generate and display an M mode image in the processors 20c and 20d is executed. That is, with respect to each of the B mode image and the M mode image, the ultrasonic scan processing for echo data and the TV scan processing for data which have been subjected to the ultrasonic scan processing are assigned to different processors 20a (BM1), 20b (BM2), 20c (MM1) and 20d (MM2) to perform the operation. As a result, each of the processors 20a (BM1), 20b (BM2) , 20c (MM1) and 20d (MM2) can successively execute signal processing having the same processing rate, and thus the processing loss is prevented.

Furthermore, the ultrasonic scan processing and the TV scan processing which are different in processing rate can be executed at a high speed in parallel in the pipelining style by using the processors 20a (BM1), 20b (BM2), 20c (MM1) and 20d (MM2), and thus the processing efficiency of the data converter 20 can be enhanced.

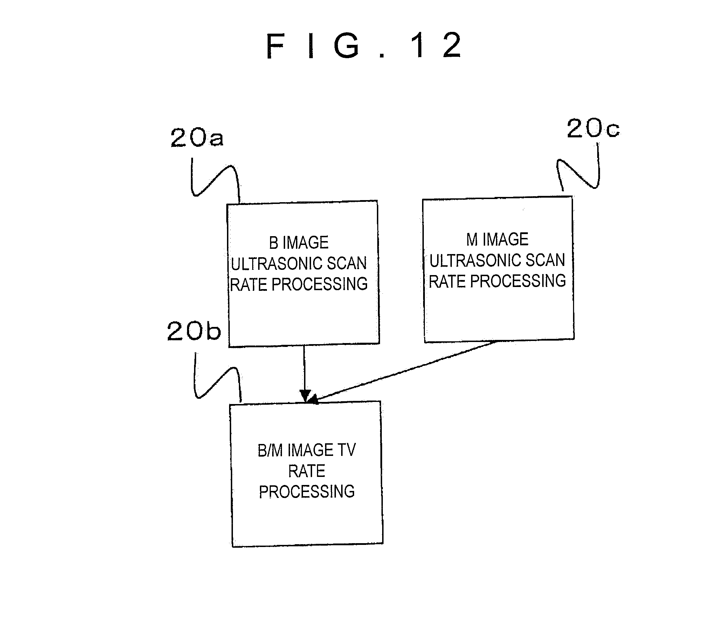

Fig. 12 shows a modification of this embodiment. For example when the processing capacity of one processor is large, the TV scan processing for the B mode image and the TV scan processing for the M mode image may be assigned to one processor 20b as shown in Fig. 12. That is, the processing rate of the ultrasonic scan processing is different between the B mode image and the M mode image, and thus the different processors 20a and 20c are assigned to them. On the other hand, the processing rate of the TV scan processing is equal between both the images, and when the processor has an extra processing capacity, the TV scan processing for both the images can be commonly assigned to the processor 20b.

### Embodiment 4

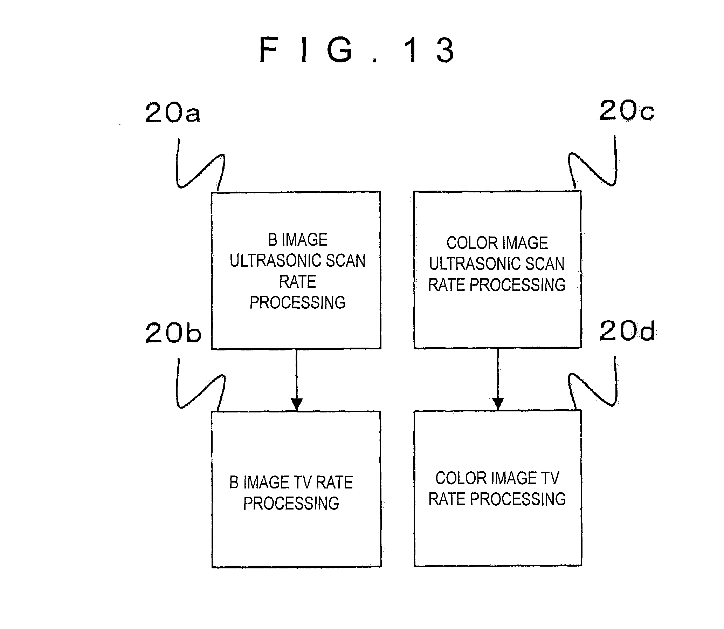

Fig. 13 is a diagram showing an example of the assignment of the processors in the color flow mode for generating and displaying a B mode image and a color image in parallel. For example, when the mode is switched from the B/M mode to the color flow mode, the assignment of the processors 20a to 20d used in the B/M mode is temporarily released, and also the memory area of the memory 20j secured in the B/M mode processing is temporarily released. In order to newly perform the operation of the color flow mode, the processors are re-assigned, and a memory area to be used in the color flow mode is secured again. Here, in accordance with the selected imaging mode (color flow mode), the data converter 20 determines the number of processors to be assigned to signal processing executed in the selected imaging mode out of the processors 20a to 20h. In this embodiment, four signal processing having different processing rates exist in the signal processing required in the color flow mode, and thus four processors are assigned to the signal processing.

This embodiment relates to an example in which the B mode image and the color image are alternately measured, and two processors are assigned to each of the B mode image and the color image in parallel to the DEMUX processing for sorting data of each mode of each image, and each processor images the B mode image and the color image.

As shown in Fig. 13, in the color flow mode, two processing of the ultrasonic scan rate processing and the TV scan rate processing exist as signal processing having different processing rates for each of the B mode image and the color image as in the case of the second embodiment. Therefore, the processor 20a (BM1) is assigned to the ultrasonic scan rate processing of the B mode image, and the processor 20b (BM2) is assigned to the TV scan rate processing of the B mode image. Furthermore, the processor 20c (CM1) is assigned to the ultrasonic scan rate processing of the color image and the processor 20d (CM2) is assigned to the TV scan rate processing of the color image.

In this embodiment, the data processing of each processor is executed as in the case of the second embodiment except that the processing required to generate and display the color image is executed in the processors 20c, 20d.
That is, with respect to each of the B mode image and the color image, the ultrasonic scan processing for echo data and the TV scan processing for data which has been subjected to the ultrasonic scan processing are assigned to the different processors 20a (BM1) , 20b (BM2), 20c (CM1) and 20d (CM2) to perform the operation. As a result, each of the processors 20a (BM1), 20b (BM2), 20c (CM1) and 20d (CM2) can successively execute signal processing having the same processing rate and thus the processing loss is prevented.

Furthermore, the ultrasonic scan processing and the TV scan processing which are different in processing rate can be executed at a high speed in parallel in the pipelining style by using the processors 20a (BM1), 20b (BM2), 20c (CM1) and 20d (CM2), and thus the processing efficiency of the data converter 20 can be enhanced.

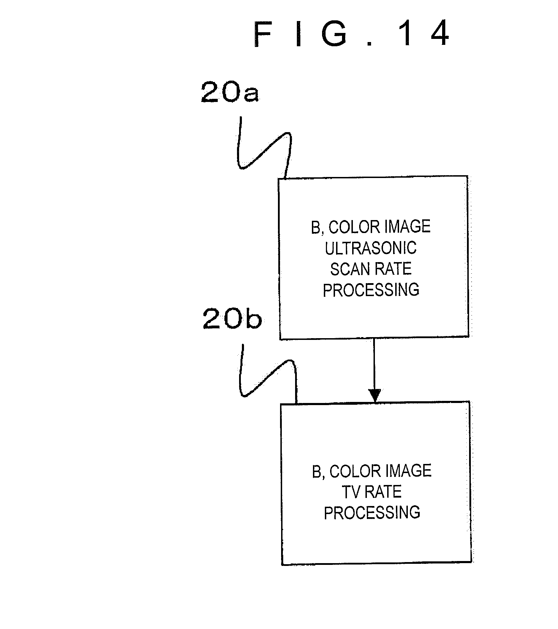

Fig. 14 is a diagram showing a modification of this embodiment. For example, in a case where the processing capacity of one processor is large, as shown in Fig. 12, when the ultrasonic scan processing for the B mode image and the ultrasonic scan processing for the color image have the same processing rate, these processing may be assigned to one processor 20a. Furthermore, when the TV scan processing for the B mode image and the TV scan processing for the color image have the same processing rate, these processing may be assigned to one processor 20b.

As described above, according to the ultrasonic diagnostic apparatus of this embodiment, with respect to the difference in processing rate of the signal processing of the echo data which is caused by the difference among various ultrasonic beam types in various imaging modes of the ultrasonic diagnostic apparatus, and the difference in processing rate caused by the ultrasonic scan processing and the TV scan processing of the echo data, the signal processing different in processing rate are assigned to different processors to perform the operation, whereby each processor can successively execute signal processing of the same processing rate, and signal processing of different processing rates can be executed at a high speed in parallel in the pipelining style. Accordingly, the processing efficiency of the processors can be enhanced.

Furthermore, the determination of the number of processors to be used and the processing contents of the processors and re-assignment of the memory area are performed in accordance with the imaging mode, whereby the resource-limited multiprocessor system can be efficiently used and thus the processing efficiency of the processors can be enhanced.
Furthermore, some preferable embodiments such as the ultrasonic diagnostic apparatus, etc. according to the present invention have been described, however, the present invention is not limited to these embodiments. It is apparent that persons skilled in the art can make various alterations or modifications within the scope of the technical idea disclosed in this application, and it is understood that they belong to the technical scope of the present invention.

### Description of Reference Numerals

10 ultrasonic diagnostic apparatus, 12 ultrasonic probe, 14 transmission/reception switching unit, 16 beam forming unit, 20 data converter, 20a to 20h processor, 24 display unit

## Claims

1. An ultrasonic diagnostic apparatus **characterized by** comprising an ultrasonic probe configured to transmit an ultrasonic beam to an examinee, and receive a reflected echo signal from the examinee; a beam forming unit configured to supply the ultrasonic probe with a driving signal for transmitting the ultrasonic beam; a data converter configured to conduct a plurality of signal processing on echo data obtained by digitizing the reflected echo signal to convert the echo data to ultrasonic image data; and a display unit configured to display an ultrasonic image based on the converted ultrasonic image data, wherein the data converter has a plurality of processors to be assigned to signal processing having different processing rates out of the plurality of signal processing.

2. The ultrasonic diagnostic apparatus according to claim 1, wherein the data converter determines the number of processors to which signal processing having different processing rates out of the plurality of signal processing is assigned.

3. The ultrasonic diagnostic apparatus according to claim 2, wherein in accordance with an imaging mode selected from a plurality of imaging modes of the ultrasonic image, the data converter determines the number of processors to which signal processing executed in the selected imaging mode out of the plurality of processors is assigned.

4. The ultrasonic diagnostic apparatus according to claim 1, wherein the data converter varies the assignment of the plurality of processors every beam type of an ultrasonic beam to be transmitted to the examinee.

5. A signal processing method for an ultrasonic diagnostic apparatus **characterized by** comprising a step that transmits an ultrasonic beam to an examinee by an ultrasonic probe, and receives a reflected echo signal from the examinee; a step that supplies the ultrasonic probe with a driving signal for transmitting an ultrasonic beam by a beam forming unit; a step that conducts a plurality of signal processing on echo data obtained by digitizing the reflected echo signal to convert the echo data to ultrasonic image data by a data converter; and a step that displays an ultrasonic image based on the converted ultrasonic image data by a display unit, wherein the data converter has a plurality of processors to be assigned to signal processing having different processing rates out of the plurality of signal processing, and the method further comprises a step that determines, from the plurality of processors, processors to be assigned to signal processing having different processing rates out of the plurality of signal processing.
